(19) Europäisches Patentamt
European Patent Office
Office européen des brevets

(11) **EP 2 438 982 A1**

(12) **EUROPÄISCHE PATENTANMELDUNG**

(43) Veröffentlichungstag:
**11.04.2012  Patentblatt 2012/15**

(51) Int Cl.:
*B01J 19/00* *(2006.01)*     *C01B 3/04* *(2006.01)*
*C01B 3/38* *(2006.01)*     *C07C 29/152* *(2006.01)*
*C07C 31/04* *(2006.01)*     *C10G 2/00* *(2006.01)*
*C10G 3/00* *(2006.01)*

(21) Anmeldenummer: **11155310.3**

(22) Anmeldetag: **22.02.2011**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **01.02.2011  EP 11152947**
**06.10.2010  PCT/EP2010/064948**

(71) Anmelder: **Silicon Fire AG**
**6045 Meggen (CH)**

(72) Erfinder: **Meyer-Pittroff, Roland**
**85354, Freising (DE)**

(74) Vertreter: **OK pat AG**
**Chamerstrasse 50**
**6300 Zug (CH)**

(54) **Verfahren zur Bereitstellung und zum Einsetzen eines Alkohols und Verwendung des Alkohols zur Wirkungsgrad- und Leistungssteigerung einer Verbrennungskraftmaschine**

(57)     Die Erfindung betrifft ein Verfahren zum Bereitstellen und Verbrauchen einer speicherbaren und transportablen, alkoholhaltigen Kühlflüssigkeit (108) mit den folgenden Schritten:
- Bereitstellen (52) eines Gases mit Kohlenstoffdioxidanteil als Kohlenstofflieferant,
- Bereitstellen eines Wasserstoffanteils,
- Bereitstellen eines Ausgangsstoffes (AS), der den Kohlenstoffdioxidanteil und Wasserstoffanteil umfasst,
- Einbringen des Ausgangsstoffes (AS) in einen Reaktor,
- Durchlaufen des Ausgangsstoffs (AS) durch eine Reaktionstrecke des Reaktors, die mindestens teilweise mit einem Katalysator bestückt ist, um dort auf synthetischem Weg die Kühlflüssigkeit (108) zu synthetisieren,
- Bereitstellen der Kühlflüssigkeit (108) an einem ausgangsseitigen Ende des Reaktors, wobei es sich bei der Kühlflüssigkeit (108) um ein Gemisch (108) aus Alkohol und Wasser handelt,
- Einsetzen der Kühlflüssigkeit (108) vor oder/und in einer Verbrennungskraftmaschine (62), die mit einem Kraftstoff beschickt wird, wobei die Kühlflüssigkeit (108) eingesetzt wird, um durch Verdampfen der Kühlflüssigkeit (108) Ansaugluft, Verdichterluft oder/und Kraftstoff-Luft-Gemisch der Verbrennungskraftmaschine (62) zu kühlen.

Fig. 4

EP 2 438 982 A1

**EP 2 438 982 A1**

**Beschreibung**

Die vorliegende Anmeldung betrifft Verfahren zur Bereitstellung und zum Einsetzen eines Alkohols. Ausserdem geht es um die Verwendung des Alkohols zur Wirkungsgrad- und Leistungssteigerung einer Verbrennungskraftmaschine, wie zum Beispiel einer Dieselkraftmaschine. Insbesondere geht es um Methanolgemische.

Die vorliegende Anmeldung beansprucht die Priorität der internationalen Patentanmeldung PCT/EP2010/064948, die am 6. Oktober 2010 unter dem Titel "VERFAHREN UND ANLAGE ZUR SYNTHESE VON KOHLENWASSERSTOFF" eingereicht wurde.

Die vorliegende Anmeldung beansprucht auch die Priorität der provisorischen Europäischen Patentanmeldung EP11152947.5, die am 1. Februar 2011 unter dem Titel "VERFAHREN ZUR BEREITSTELLUNG UND ZUM EINSETZEN EINES ALKOHOLS UND VERWENDUNG DES ALKOHOLS ZUR LEISTUNSSTEIGERUNG EINER VERBRENNUNGS-KRAFTMASCHINE" eingereicht wurde.

Kohlenstoffdioxid $CO_2$ (meist Kohlendioxid genannt) ist eine chemische Verbindung aus Kohlenstoff und Sauerstoff. Kohlendioxid ist ein farbund geruchloses Gas. Es ist mit einer geringen Konzentration ein natürlicher Bestandteil der Luft und entsteht in Lebewesen bei der Zellatmung, aber auch bei der Verbrennung von kohlenstoffhaltigen Substanzen bei ausreichender Anwesenheit von Sauerstoff. Seit Beginn der Industrialisierung steigt der $CO_2$-Anteil in der Atmosphäre deutlich an. Hauptursache hierfür sind die vom Menschen verursachten - sogenannten anthropogenen - $CO_2$-Emissionen. Das Kohlendioxid in der Atmosphäre absorbiert einen Teil der Wärmestrahlung. Diese Eigenschaft macht Kohlendioxid zu einem so genannten Treibhausgas (THG) und zu einem der Mitverursacher des globalen Treibhauseffekts.

Aus diesen und auch aus anderen Gründen wird zur Zeit in verschiedenste Richtungen geforscht und entwickelt, um einen Weg zu finden, um die anthropogenen $CO_2$-Emissionen zu reduzieren. Besonders im Zusammenhang mit der Energieerzeugung, die häufig durch das Verbrennen fossiler Energieträger, wie Kohle, Öl oder Gas, erfolgt, aber auch mit anderen Verbrennungsprozessen, zum Beispiel der Müllverbrennung, besteht ein großer Bedarf zur Reduktion der $CO_2$-Emission. Es werden pro Jahr über 20 Milliarden Tonnen $CO_2$ durch solche Prozesse in die Atmosphäre abgegeben.

Es wird als Problem angesehen, dass bei der Verbrennung von fossilen Energieträgern $CO_2$ entsteht. Ausserdem werden die fossilen Ressourcen, die endlich sind, unwiderruflich verbraucht. Insbesondere die Mobilität ist mit größeren Emissionen verbunden. Daher wird in verschiedenste Richtungen geforscht, um den Verbrauch von Fahrzeugen zu senken oder um Fahrzeuge zu entwickeln, die komplett aus regenerativen Energieformen angetrieben werden.

Es ist bekannt, dass die Ansaugluft oder das Kraftstoff-Luft-Gemisch vor der Verdichtung im Ansaugtrakt einer Verbrennungskraftmaschine gekühlt werden kann. Das führt zur Erhöhung der Dichte des Gases und damit zur Erhöhung des Massenstromes durch die Maschine und zur Möglichkeit, mehr Brennstoff in der vergrößerten Luftmasse zu verbrennen, und damit zur Erhöhung der spezifischen Leistung.

Der Arbeitsprozess und damit auch die Verbrennung im Verbrennungsraum einer Verbrennungskraftmaschine beginnen bei niedrigerer Temperatur. Bei (wegen der thermischen Belastung) vorgegebener Verbrennungs-Endtemperatur (vor allem bei Gasturbinen wegen der Schaufelfestigkeit) kann bei Verbrennungsbeginn mit niedrigerer Temperatur ebenfalls mehr Wärme zugeführt werden, was wiederum zur Erhöhung der spezifischen Leistung führt.

Stand der Technik ist bei fast allen modernen Diesel-Motoren und bei vielen Otto-Motoren die Vorverdichtung der Verbrennungsluft bzw. des Kraftstoff-Luft-Gemisches vor dem Verbrennungsraum mit einem speziellen Verdichter (auch Lader oder Kompressor genannt) aus den bereits erwähnten Gründen. Üblich dafür ist heute der Einsatz eines Radialverdichters, der von einer Radial-Abgasturbine angetrieben wird (Abgasturbolader genannt). Radialverdichter und Radial-Abgasturbine sind auf einer gemeinsamen Welle angeordnet. Durch diese Verdichtung erwärmt sich das Gas, respektive die die Ladeluft, polytrop und wird dem Stand der Technik entsprechend mit einem z.B. durch Außenluft gekühlten Wärmetauscher (Zwischenkühler oder Intercooler genannt) zurück gekühlt, um Leistung und Wirkungsgrad des Motors zu erhöhen.

Die höhere spezifische Leistung der Maschine bei sonst gleich bleibenden Verlusten führt zusätzlich zur Erhöhung des Wirkungsgrades, was zum Beispiel bei Verbrennungsmotoren durch Verkleinern der Maschine genutzt wird ("downsizing").

Der Zwischenkühler verursacht Bauaufwand mit entsprechenden Kosten und Platzbedarf sowie wegen seines Strömungswiderstandes (Druckverlustes) auch Verluste bezüglich Leistung und Wirkungsgrad.

Es ist auch bekannt, dass man durch das Einspritzen von Wasser oder Wasser-Alkohol-Gemischen die Ansaugluft bzw. das Kraftstoff-Luft-Gemisch eines Motors abkühlen kann und zwar sowohl vor der Verdichtung im Ansaugtrakt als auch nach der Vorverdichtung anstelle des Zwischenkühlers oder auch nach dem Zwischenkühler. Durch das Einspritzen einer solchen Flüssigkeit in die Ansaugluft bzw. in das Kraftstoff-Luft-Gemisch können Leistungs- und Wirkungsgradgewinne ohne Druckverlust erzielt werden. Das liegt daran, dass das Wasser oder das Wasser-Alkohol-Gemisch verdampft und entsprechend seiner Verdampfungswärme die Ansaugluft bzw. das Kraftstoff-Luft-Gemisch abkühlt.

Wenn diese Flüssigkeit brennbar ist, wie dies bei einem Wasser-Alkohol-Gemisch der Fall ist, nimmt sie am nachfolgenden Verbrennungvorgang in der Verbrennungskraftmaschine unter Abgabe zusätzlicher mechanischer Leistung teil.

Man kann je nach Ausgestaltung der entsprechenden Aggregate einer solchen Verbrennungskraftmaschine und je nach Einsatz von Wasser oder Wasser-Alkohol-Gemischen unterschiedliche Effekte erzielen. So können zum Beispiel die Gesamtleistung und die innere Kühlung der Verbrennungskraftmaschine erhöht werden. Dadurch wird zum Beispiel das "Downsizing" der Verbrennungskraftmaschine ermöglicht, was zur Verringerung der Verluste und auch zu einer Reduktion der Emissionen beitragen kann.

Wenn Alkohol zum Einsatz kommt, dann verbrennt dieser Alkohol zusammen mit dem Kraftstoff (Primärkraftstoff) in der Verbrennungskraftmaschine. Falls der Alkohol aus fossilen Rohstoffen gewonnen wurde, dann führt das Verbrennen des Alkohols jedoch zur zusätzlichen $CO_2$-Emission.

Es wird daher untersucht, ob und inwieweit man Alkohol aus biogenen Ressourcen erzeugen und in Fahrzeugmotoren einsetzen kann. Der Einsatz von $CO_2$-neutralem Alkohol würde die klimawirksame $CO_2$-Emission des Fahrzeugmotors reduzieren. Der Hauptaugenmerk ist hier auf Methanol, Ethanol und Butanol gerichtet. Leider zeigt sich, dass die Produktion von $CO_2$-neutralem Alkohol verschiedene andere Nachteile mit sich bringt. So steht zum Beispiel die Produktion dieser Alkohole häufig in unmittelbarer Konkurrenz mit der Nahrungsmittelproduktion, oder die Kosten für den $CO_2$-neutralen Alkohol liegen erheblich über den Kosten fossiler Kraftstoffe.

Auch andere alternative alkoholbasierte Kraftstoffe sind oft teurer als die fossilen Kraftstoffe. Dies liegt zum Teil daran, dass bei der Produktion von $CO_2$-neutralem Alkohol alle Kosten, inklusive der Umweltkosten, mit eingerechnet werden, wohingegen fossile Kraftstoffe heute immer noch ohne wirkliche Berücksichtigung der sogenannten externen Kosten angeboten werden.

Methanol ist ein besonders vorteilhafter Alkohol, da er der einfachste Alkohol ist, den es gibt. Methanol wird jedoch bisher in den meisten Fällen aus fossilen Rohstoffen, zum Beispiel aus Erdgas, hergestellt. Es sind zahlreiche Verfahren und Reaktoren zur Herstellung von Methanol bekannt. Im Folgenden sind entsprechende beispielhafte Patentanmeldungen und Patente genannt:

- EP 0 790 226 B1;
- WO 2010/037441 A1;
- EP 4 483 919 A2.

Es besteht für die eingangs erwähnten Anwendungen der Bedarf für die Bereitstellung eines Alkohol-Wasser-Gemischs, das einerseits als Kühlflüssigkeit geeignet ist und das andererseits $CO_2$-neutral und günstig in der Herstellung ist. Ausserdem soll der Alkohol nicht mit der Nahrungsmittelproduktion in Konkurrenz stehen.

Es stellt sich nun die Aufgabe, ein entsprechendes Verfahren zur Bereitstellung einer alkoholhaltigen Kühlflüssigkeit zu entwickeln, das ökologisch und ökonomisch sinnvoll ist. Die Kühlflüssigkeit soll optimal zum Kühlen der Ansaugluft bzw. des Kraftstoff-Luft-Gemisches einer Verbrennungskraftmaschine (z.B. eines Auto- oder Schiffsmotors) geeignet sein.

Gemäss Erfindung wird daher eine neue Verfahrenskette vorgeschlagen, bei der es um das Bereitstellen und Verbrauchen einer speicherbaren und transportablen alkoholhaltigen Kühlflüssigkeit geht. Das Verfahren umfasst die folgenden Schritten:

- Bereitstellen eines Gases mit Kohlendioxidanteil ($CO_2$) als Kohlenstofflieferant,
- Bereitstellen eines Wasserstoffanteils ($H_2$),
- Bereitstellen eines Ausgangsstoffes, der den Kohlendioxidanteil und Wasserstoffanteil umfasst,
- Einbringen des Ausgangsstoffes in einen Reaktor,
- Durchlaufen des Ausgangsstoffs durch eine Reaktionstrecke des Reaktors, die mindestens teilweise mit einem Katalysator bestückt ist, um in einem synthesekatalytischen Verfahren die alkoholhaltige Kühlflüssigkeit zu synthetisieren,
- Bereitstellen der alkoholhaltigen Kühlflüssigkeit an einem ausgangsseitigen Ende des Reaktors, wobei diese Kühlflüssigkeit ein Gemisch aus einem Alkoholanteil und einem Wasseranteil ist,
- Einsetzen dieser alkoholhaltigen Kühlflüssigkeit in einer Verbrennungskraftmaschine, die mit Luft oder einem Kraftstoff-Luft-Gemisch beschickt wird, wobei die Luft oder das Kraftstoff-Luft-Gemisch durch Verdampfen der alkoholhaltigen Kühlflüssigkeit (vor-)gekühlt wird.

Insbesondere geht es hier um Wärmekraftmaschinen mit innerer Verbrennung, dass heißt es geht um Verbrennungskraftmaschinen, die Luft oder ein Kraftstoff-Luft-Gemisch gasförmig ansaugen, verdichten und nach der Verdichtung diesem Arbeitsmedium Wärme zuführen durch Verbrennung innerhalb des Arbeitsmediums. Solche Verbrennungskraftmaschinen sind Otto-und Diesel-Motoren und offene Gasturbinen. Die Erfindung lässt sich also vorzugsweise auf Otto-Motoren, Diesel-Motoren und offene Gasturbinen anwenden.

Die Erfindung setzt bewusst auf Kohlendioxid und Wasserstoff als Ausgangsstoffe, da das Kohlendioxid auf diesem Wege "rezykliert" werden und als Kohlenstofflieferant dienen kann. Wenn man den Wasserstoff aus regenerativer Energie erzeugt und das Kohlendioxid aus Abgasen gewonnen oder aus Biomasse erzeugt wird, so wird die alkoholhaltige Flüssigkeit, die aus diesen Ausgangsstoffen auf katalytischem Weg synthetisiert wird, als $CO_2$-neutral angesehen. Ausserdem hat dieser Weg den Vorteil, dass er z.B. bei Synthese von Methanol ein Methanol-Wasser-Gemisch liefert, das direkt zum Ansaugkühlen oder Zwischenkühlen geeignet ist. Die Zusammensetzung der alkoholhaltigen Flüssigkeit ist aus chemisch-physikalischer Sicht ideal, um als Fluid zum Ansaugkühlen oder Zwischenkühlen eingesetzt zu werden, und sie zeichnet sich dadurch aus, dass sie auch aus umwelttechnischer Sicht Vorteile hat.

Insbesondere, wenn es um das Zwischenkühlen eines Diesel-Motors geht, könnte ein Mischbetrieb, bei dem einerseits Dieselkraftstoff und andererseits als Kühlflüssigkeit reiner Alkohol (der sich beim Verbrennen benzinähnlich verhält) eingesetzt wird, die Lebensdauer des Diesel-Motors negativ beeinträchtigen. Daher ist besonders in diesen Fällen der Einsatz einer Kühlflüssigkeit vorteilhaft, die außer Methanol zur Hälfte bis zu zwei Dritteln aus Wasser besteht.

Anders als bei bisherigen Methanol-Produktionsverfahren kann hier das Produkt des Methanol-Syntheseprozesses, bestehend aus Methanol und Wasser, unmittelbar als Kühlflüssigkeit betrachtet und eingesetzt werden. Es braucht keinen weiteren Energieaufwand zum Destillieren des Produkts des Syntheseprozesses. Der Energieaufwand zum Destillieren, der typischerweise betrieben wird, um reinen Alkohol zu erhalten, führt zu einer Verteuerung des Alkohols. Gemäss Erfindung kommt das unmittelbar erzeugte Alkohol-Wasser-Gemisch zum Zwischenkühlen oder Ansaugkühlen zum Einsatz, das durch die katalytische Synthese von Kohlendioxid und Wasserstoff bereit gestellt wird.

Vom Stand der Technik unterscheidet sich das Verfahren unter anderem dadurch, dass keine fossilen Rohstoffe, wie zum Beispiel Methangas, zum Einsatz kommen, um den Alkohol zu erzeugen. Ausserdem geht das erfindungsgemässe Verfahren den Weg über die katalytische Synthese aus Kohlendioxid und Wasserstoff, statt über Kohlenmonoxid und Wasserstoff. Die katalytische Synthese aus Kohlendioxid und Wasserstoff liefert nämlich bereits eine nahezu ideale Zusammensetzung des Produktes als Kühlflüssigkeit, wie bereits beschrieben. Das unmittelbare Syntheseprodukt der katalytischen Synthese, das hier als alkoholhaltige Kühlflüssigkeit bezeichnet wird, wird zur Ansaugkühlung oder Zwischenkühlung eingesetzt.

Gemäss Erfindung wird $H_2$- und $CO_2$-haltiges Synthesegas effizient und wirtschaftlich sinnvoll zu einer alkoholhaltigen Kühlflüssigkeit, die z.B. Methanol und/oder Ethanol enthält, umgesetzt.

Gemäß Erfindung wird Kohlendioxid als Kohlenstofflieferant eingesetzt. Das Kohlendioxid wird mit dem Wasserstoffanteil in Anwesenheit eines Katalysators zur Reaktion gebracht, um diese Gase zu einer Kühlflüssigkeit, vorzugsweise einem Wasser-Alkohol-Gemisch, und besonders vorzugsweise einem Methanol-Wasser-Gemisch umzusetzen.

Vorzugsweise wird Kohlendioxid aus einem Verbrennungsprozess oder einem Oxidationsprozess von Kohlenstoff oder Kohlenwasserstoffen mittels $CO_2$-Abscheidung entnommen. $CO_2$ kann zum Beispiel über eine Pipeline oder aber auch in Stahlflaschen oder Tanks bereit gestellt werden. Es kann aber auch Kohlendioxid aus einem Fahrzeug, wie zum Beispiel einem Schiff, eingesetzt werden.

Der Wasserstoff kann über eine Pipeline oder aber auch in Stahlflaschen oder Tanks bereit gestellt werden. Vorzugsweise wird der Wasserstoff jedoch vor Ort mittels Wasserelektrolyse hergestellt. Alternativ kann der Wasserstoff auch durch eine Reduktionsreaktion von Wasser mit elementarem Silizium oder einem anderen elementaren Metall erzeugt werden.

Kohlendioxid als Kohlenstofflieferant kann gemäß Erfindung auch aus Roherdgas entnommen werden, das je nach Erdgasquelle über 10% Kohlendioxid-Anteil aufweisen kann. Kohlendioxid kann auch aus industriellen Prozessen, z.B. des Kalkbrennens oder des Kalzinierens zu Soda, stammen.

Das erfindungsgemäße Verfahren zur Bereitstellung der Kühlflüssigkeit wird so gesteuert und die einzelnen Prozesse werden so miteinander "verknüpft", dass

- der Gesamtertrag und die Qualität der Kühlflüssigkeit ideal für den Einsatzzweck ist,
- und/oder die $CO_2$-(Gesamt-)Emissio möglichst minimal wird,
- und/oder eine möglichst konstante und langzeitige Anlagenauslastung erzielt wird,
- und/oder die produktspezifischen Investitions- und Betriebskosten möglichst minimal werden.

Vorzugsweise wird regenerative elektrische Energie zum Bereitstellen der Kühlflüssigkeit eingesetzt.

Mit einer entsprechenden Anlage wird vorzugsweise ein Methanol-Wasser-Gemisch als speicher- und transportierbare Kühlflüssigkeit hergestellt. D.h., es werden die erneuerbaren Energien auf chemischem Wege in eine unkritische und relativ einfach speicher- und transportierbare Kühlflüssigkeit überführt.

Die Produktion der Kühlflüssigkeit als relativ einfach speicher- und transportierbares Gemisch kann jederzeit heruntergefahren oder gar unterbrochen werden. Die verfahrenstechnischen Anlagenteile zur Herstellung des Gemischs können relativ einfach und schnell heruntergefahren oder abgeschaltet werden oder in Abhängigkeit von der Netzfrequenz betrieben werden. Hier liegt die Entscheidungshoheit im Verantwortungsbereich des Betreibers der Anlage. Dadurch ergibt sich die Möglichkeit, die Anlage an der Stabilisierung und/oder der Frequenzregelung des elektrischen Verbundnetzes zu beteiligen.

Bevorzugte Ausführungsformen der Erfindung basieren auf der Wasserstofferzeugung mit Hilfe elektrischer Energie, die weitmöglich regenerativ erzeugt wird und z.B. aus Wind-, Wasser-, Geothermie- und/oder Solarkraftwerken stammt. Wasserstoff, der z.B. vor Ort per Elektrolyse oder durch den Einsatz von elementarem Silizium oder anderen Metallen erzeugt wird, braucht also nicht gelagert oder hoch verdichtet oder tief gekühlt verflüssigt und über größere Strecken transportiert zu werden, sondern dient als Zwischenprodukt, das vorzugsweise am Standort seiner Erzeugung unmittelbar oder zeitnah der vorgenannten Reaktion zur Erzeugung der Kühlflüssigkeit zugeführt wird.

Einem energieumwandelnden Prozess, bei dem regenerative Energie in elektrische Energie umgewandelt wird, folgen je nach Ausführungsform der Erfindung z.B. stoffumwandelnde (chemische) Prozesse, nämlich die intermediäre Bereitstellung von Wasserstoff und die Umwandlung des Wasserstoffs zusammen mit dem Kohlendioxid zu der alkoholhaltigen Kühlflüssigkeit, vorzugsweise einer Kühlflüssigkeit, die Methanol enthält.

Das entsprechende Alkohol-Wasser-Gemisch kann aber gemäss Erfindung auch unter Einsatz eines intelligenten Energiemixes (wie z.B. in der internationalen Patentanmeldung WO2010069622A1 beschrieben) aus fossiler und regenerativer Energie erzeugt werden.

Unter Beachtung entsprechender energietechnischer, anlagentechnischer und wirtschaftlicher Vorgaben, zusammen mit der Forderung nach schonender Nutzung aller stofflichen, energetischen und ökonomischen Ressourcen, werden gemäß Erfindung ein neues, energietechnisch relevantes Verfahren und eine entsprechende Verwendung bereitgestellt.

Es wird als ein Vorteil der Erfindung angesehen, dass die Erniedrigung der mittleren Verbrennungstemperatur einer Verbrennungskraftmaschine zu einer Verringerung der Stickoxid (NOx) Emissionen führt.

Es ist auch ein Vorteil der Erfindung, dass eine Kühlung des Arbeitsmediums während der Verdichtung zur Erhöhung von spezifischer Leistung und Wirkungsgrad der Verbrennungskraftmaschine führt.

Apparativ/konstruktiv wird die Kühlung während der Verdichtung am besten bei mehrstufiger Verdichtung realisiert, z.B. bei einer zweistufigen Verdichtung des Arbeitsmediums der Verbrennungskraftmaschine durch Lader/Kompressor und Kolben im Zylinder oder bei Gasturbinen mit Nieder- und Hochdruck-Verdichter. Die Kühlung zwischen einzelnen Verdichtungsstufen, die hier als Zwischenkühlung bezeichnet wird, kann neben der Verringerung der Verdichtungsarbeit ebenfalls zu den oben erwähnten Vorteilen führen, die aus der Erhöhung der Dichte des Arbeitsmediums in den nachfolgenden Stufen und der Temperaturerniedrigung des Verbrennungsbeginns zusammenhängen. Daraus ergeben sich Leistungs- und Wirkungsgrad-Erhöhungen und eine NOx-Emissionsverminderung.

Es geht bei der Erfindung also um die Kühlung eines Arbeitsmediums, d.h. es geht im Speziellen um Verfahren und Vorrichtungen zur Ansaugkühlung und Zwischenkühlung einer Verbrennungskraftmaschine.

Weitere vorteilhafte Ausführungsformen sind der Beschreibung, den Figuren und den abhängigen Ansprüchen zu entnehmen.

In den Zeichnungen sind verschiedene Aspekte der Erfindung schematisch dargestellt.

Fig. 1:  zeigt ein Schema, das die grundlegenden Schritte des Verfahrens, gemäß einer der eingangs erwähnten internationalen Patentanmeldungen, respektive einer entsprechenden Silicon-Fire Anlage wiedergibt;

Fig. 2:  zeigt ein Schema, das die grundlegenden Schritte des Verfahrens gemäß Erfindung, respektive einer entsprechenden Silicon-Fire Anlage wiedergibt;

Fig. 3:  zeigt eine seitliche Außenansicht eines Reaktors, der in einem erfindungsgemässen Verfahren eingesetzt werden kann;

Fig. 4:  zeigt eine stark schematisierte Ansicht eines Gesamtverfahrens gemäss Erfindung;

Fig. 5:  zeigt eine schematische Ansicht einer Vorrichtung, die einen Verdichter mit Zwischenkühlung umfasst;

Fig. 6:  zeigt eine schematische Ansicht einer Vorrichtung, die einen Verdichter, eine Wärmeumwandlungsanlage, zwei Tanks und einen Mikroreaktor umfasst.

Der Begriff Kühlflüssigkeit wird hier verwendet für flüssige Gemische, die direkt für die Ansaug- oder/und Zwischenkühlung eingesetzt werden können. Hier geht es insbesondere um Methanol-Wasser- oder Ethanol-Wasser-Gemische 108, bzw. um methanol- oder ethanolhaltige Kühlflüssigkeit. Es können auch Methanol-Ethanol-Wasser-Gemische 108 in allen Ausführungsformen eingesetzt werden.

Der Begriff Gemisch 108 wird hier verwendet, da das Produkt, das am Ausgang 23 eines Reaktors 10 bereit gestellt wird, nicht zu hundert Prozent aus Alkohol besteht. Es handelt sich vielmehr um ein sogenanntes physikalisches Gemisch aus Methanol und Wasser, Ethanol und Wasser, oder Methanol, Ethanol und Wasser. Die folgenden Beispiele beziehen sich auf Methanol-Wasser-Gemische 108, können aber auch auf die anderen erwähnten Gemische 108 übertragen werden. Das Methanol und Ethanol stammt vorzugsweise aus unterschiedlichen Reaktoren oder Anlagen und kann dann zu einem Methanol-Ethanol-Wasser-Gemisch 108 zusammen gebracht werden.

Der Begriff Wärmekraftmaschine mit innerer Verbrennung 62 wird hier für Verbrennungskraftmaschinen, d.h. für Otto-Motoren, Diesel-Motoren und auch (offene) Gasturbinen, verwendet.

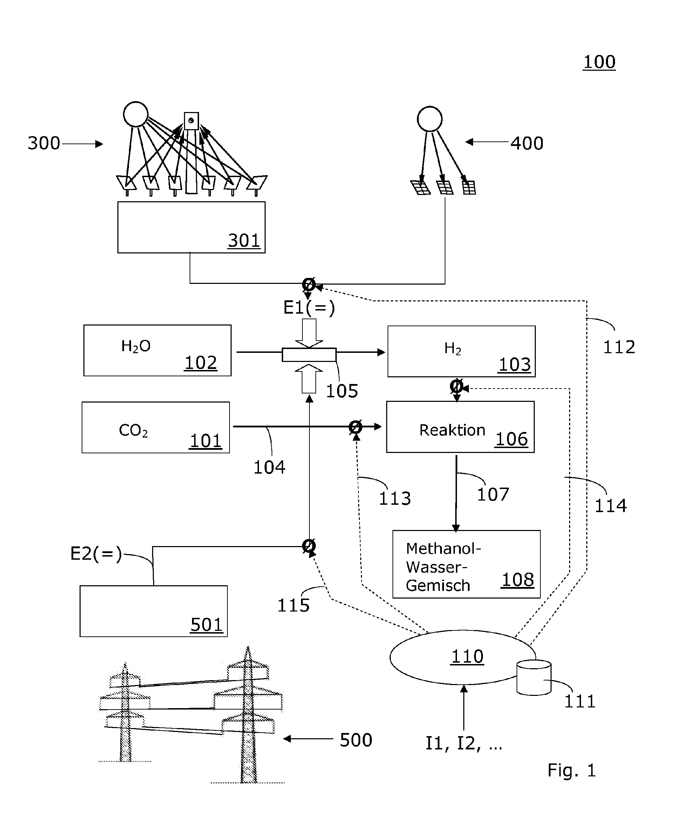

Fig. 1 zeigt in einer schematischen Blockdarstellung die wichtigsten Bausteine/Komponenten, respektive Verfahrensschritte, einer Silicon-Fire Anlage 100 gemäß einer der eingangs erwähnten internationalen Patenanmeldungen. Diese Anlage 100 ist so ausgelegt, dass ein Verfahren zum Bereitstellen speicherbarer und transportabler alkoholhaltiger Kühlflüssigkeit 108 ausgeführt werden kann. Das entsprechende Verfahren basiert auf den folgenden grundlegenden Schritten.

Es wird Kohlenstoffdioxid 101 als Kohlenstofflieferant bereitgestellt. Die für das Erzeugen von Wasserstoff 103 erforderliche elektrische Gleichstromenergie E1 wird hier weitmöglich mittels erneuerbarer Energietechnik erzeugt und der Silicon-Fire Anlage 100 zur Verfügung gestellt. Besonders als erneuerbare Energietechnik geeignet sind Solarthermieanlagen 300 und Photovoltaikanlagen 400, die auf Solarmodulen basieren. Es kann z.B. auch Wasser- oder Windkraft oder Geothermie eingesetzt werden.

Es wird gemäß Fig. 1 eine Wasserelektrolyse 105 unter Einsatz der elektrischen Gleichstromenergie E1 durchgeführt, um Wasserstoff 103 als Zwischenprodukt zu erzeugen.

Bei der Silicon-Fire Anlage 100 wird vorzugsweise eine wirtschaftlich und ökologisch optimale Kombination regenerativer Stromversorgung (z.B. durch die Anlagen 300 und/oder 400) und konventioneller Stromversorgung, hier durch einen Teil eines Verbundnetzes 500 dargestellt, realisiert. Diese Silicon-Fire Anlage 100 sieht daher vor, die regenerative elektrische Energie E1 weitgehend direkt entsprechend ihrem Anfall für chemische Reaktionen (hier die Elektrolysereaktion 105) zu nutzen und damit chemisch zu binden und zu speichern. Ein weiterer Anteil der benötigten Energie wird hier beispielsweise aus dem Verbundnetz 500 bezogen. Dieser Anteil wird in Gleichstrom(energie) E2 umgewandelt. Zu diesem Zweck kommt ein entsprechender Umwandler 501 zum Einsatz, wie in Fig. 1 in schematischer Form angedeutet. Die entsprechenden Anlagenteile oder -komponenten werden hier auch als Energieversorgungsanlage 501 bezeichnet.

Mittels einer intelligenten Anlagensteuerung 110 wird die Energieversorgung der Anlage 100 nach Fig. 1 gesteuert und geregelt. Es wird im Prinzip der jeweils momentan verfügbare überschüssige Energieanteil E2 aus dem Verbundnetz 500 bezogen, während der andere Energieanteil (hier E1) soweit möglich aus einem (Anlage-bezogenen) Solarkraftwerk 300 und/oder 400 (und/oder aus einem Windkraftwerk und/oder aus einem Wasserkraftwerk und/oder aus einem Geothermiekraftwerk) bezogen wird. Dieses Prinzip ermöglicht es dem Betreiber einer Silicon-Fire Anlage 100, zusätzliche technische und wirtschaftliche Parameter bei der Steuerung der Anlage 100 einzubeziehen. Bei diesen Parametern handelt es sich um sogenannte Input-Größen I1, I2, usw., die von der Steuerung 110 in Entscheidungen einbezogen werden. Ein Teil der Parameter kann innerhalb der Steuerung 110 in einem Parameterspeicher 111 vorgegeben werden. Ein anderer Teil der Parameter kann von außen kommen. Hier können zum Beispiel Preis- und/oder Verfügbarkeitsinformationen vom Betreiber des Verbundnetzes 500 eingehen. Durch die Steuerung 110 kann das erfindungsgemässe Verfahren in der Anlage 100 so geführt werden, dass die Kühlflüssigkeit 108, die ausgangsseitig bereit gestellt wird, die gewünschten Anforderungen in Bezug auf das Mischungsverhältnis und/oder die $CO_2$-Neutralität erfüllt.

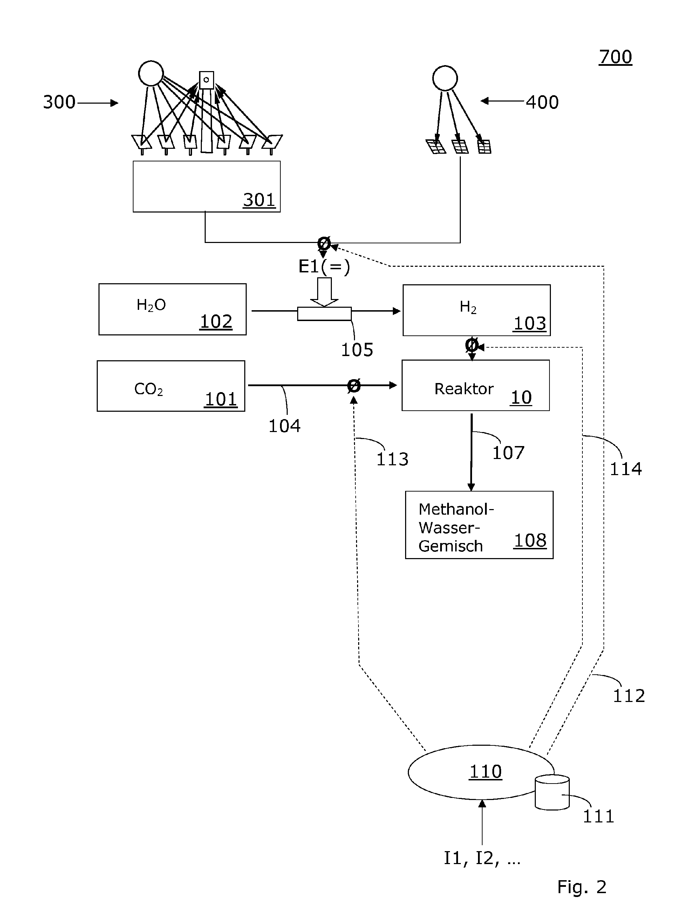

In Fig. 2 ist eine weitere Anlage 700 schematisch dargestellt, die eingesetzt werden kann, um das erfindungsgemässe Verfahren auszuführen. Ein Teil dieser Anlage 700 entspricht der Anlage 100 nach Fig. 1. Es wird daher auf die vorausgehende Beschreibung der entsprechenden Elemente verwiesen.

Es wird auch bei dieser Anlage 700, wie beschrieben, durch eine Wasserelektrolyse 105 hochreiner Wasserstoff 103 erzeugt, der hier z.B. zu einem Methanol-Wasser-Gemisch 108 umgesetzt wird. Die Energie hierzu stammt bei dieser Ausführungsform ganz oder weitestgehend (vorzugsweise zu mehr als 80 %) aus regenerativen Energiequellen 300 und/oder 400 (oder aus anderen regenerativen Energiequellen).

Es können eine Reihe von Steuer- oder Signalleitungen vorgesehen sein, wie anhand der beispielhaft gezeigten Leitungen 112, 113, 114 und 115 dargestellt. Diese Leitungen 112, 113, 114 und 115 steuern Energie- oder Massenströme der Anlage 100 oder 700.

In der Anlagensteuerung 110 sind sogenannte software-basierte Entscheidungsprozesse implementiert. Ein Prozessor der Steuerung 110 führt eine Steuerungssoftware aus und fällt unter Berücksichtigung von Parametern programmierte Entscheidungen. Diese Entscheidungen werden in Schalt- oder Steuerungsbefehle umgesetzt, die zum Beispiel über Steuer- oder Signalleitungen 112, 113, 114, 115 die Steuerung/Regelung von Energie- und Massenströmen bewirken. Durch die Steuerung 110 kann das Verfahren in der Anlage 100 so geführt werden, dass die Kühlflüssigkeit 108, die ausgangsseitig bereit gestellt wird, die gewünschten Anforderungen in Bezug auf das Mischungsverhältnis und/ oder die $CO_2$-Neutralität erfüllt.

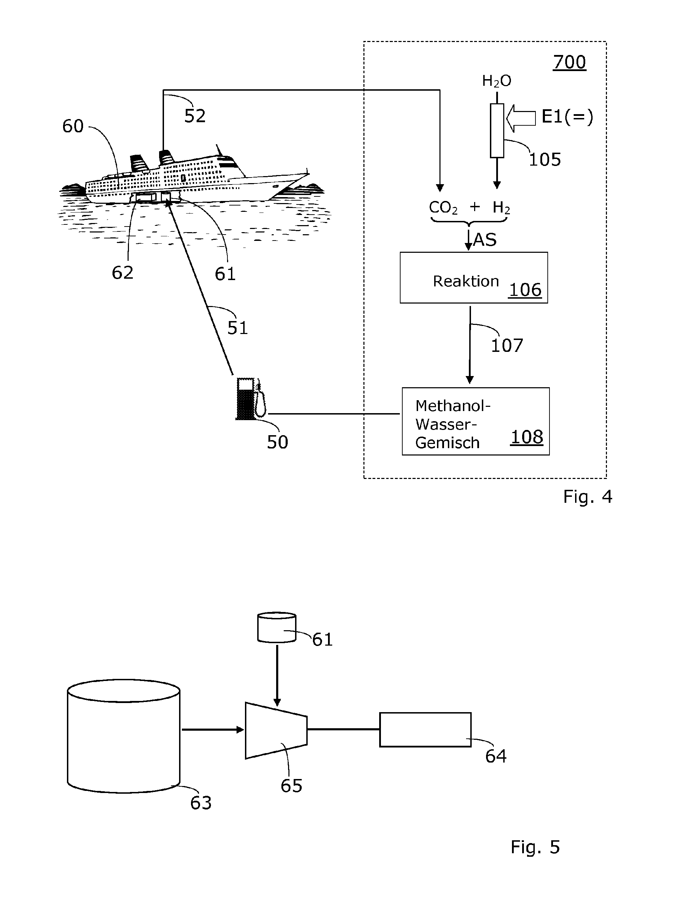

Gemäß Erfindung wird Kohlendioxid 101 als gasförmiger Kohlenstofflieferant eingesetzt, wie in Fig. 1, Fig. 2 und Fig. 4 schematisch angedeutet. Vorzugsweise wird das Kohlendioxid 101 aus einem Verbrennungsprozess oder einem Oxidationsprozess über $CO_2$-Abscheidung (z.B. eine Silicon-Fire Rauchgasreinigungsanlage) entnommen. Das Kohlendioxid 101 kann aber auch von Roherdgas abgetrennt und bereitgestellt werden. Das Kohlendioxid 101 kann auch aus anderen Quellen kommen. Vorzugsweise wird das Kohlendioxid 101 über eine Pipeline, eine Stahlflasche oder einen Tank bereit gestellt. Fig. 4 zeigt in schematischer Form, dass das $CO_2$ aus den Abgasen eines Fahrzeugs, z.B. aus einem Schiff 60, das im Hafen vor Anker liegt und dessen Aggregate laufen, stammen kann.

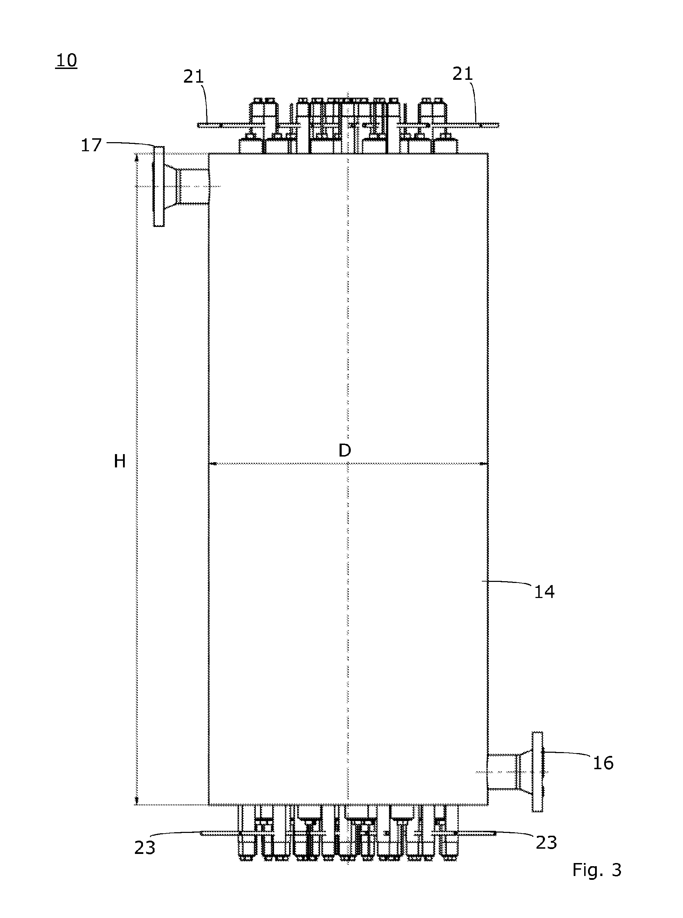

Weiterhin wird bei der in Fig. 2 gezeigten Anlage 700 elektrische Gleichstromenergie E1 bereitgestellt (das gleiche gilt auch für die Anlage in Fig. 4). Die Gleichstromenergie E1 wird vorzugsweise weitgehend regenerativ (z.B. durch eine der Anlagen 300 und/oder 400 in Fig. 2) erzeugt. Die Gleichstromenergie E1 wird bei der gezeigten Anlage 700 zum Durchführen einer Wasserelektrolyse eingesetzt, um Wasserstoff 103 als Zwischenprodukt zu erzeugen. Die Elektrolyseanlage, respektive das Durchführen einer solchen Elektrolyse, ist in Fig. 1, Fig. 2 und Fig. 4 durch das Bezugszeichen 105 gekennzeichnet. Das Kohlendioxid 101 wird mit dem Wasserstoff 103 zusammen geführt. Das entsprechende Gas wird hier als Ausgangsstoff AS bezeichnet. Der Ausgangsstoff AS wird zur Reaktion (Methanolsynthese in einem Reaktor 10, wie z.B. in Fig. 3 gezeigt) gebracht, um die gasförmigen (Zwischen-)Produkte 101, 103 z.B. zu einem Methanol-Wasser-Gemisch 108 umzusetzen. Die Reaktion wird in dem Reaktor 10 durchgeführt. Die Entnahme, respektive das Bereitstellen des Methanol-Wasser-Gemischs 108, ist in Fig. 1, Fig. 2 und Fig. 4 durch das Bezugszeichen 107 gekennzeichnet.

Im Folgenden werden weitere grundlegende Details des erfindungsgemässen Verfahrens und der entsprechenden Silicon-Fire Anlage 700 beschrieben.

Um Wasserstoff 103 als Zwischenprodukt erzeugen zu können, eignet sich eine Wasserelektrolyse unter Einsatz von Gleichstrom E1. Der benötigte Wasserstoff 103 wird in einer Elektrolyseanlage 105 durch die Elektrolyse von Wasser $H_2O$ nach folgender Gleichung hergestellt:

$$H_2O - 286{,}02\ kJ\ =\ H_2 + 0{,}5\ O_2. \qquad\qquad \text{(Reaktion 1)}$$

Die benötigte (elektrische) Energie E1 für diese Reaktion von 286,02 kJ/mol entspricht 143010 kJ pro kg $H_2$.

Die Synthese des Methanol-Wasser-Gemischs 108 ($CH_3OH$) kann in dem Reaktor 10 der Silicon-Fire Anlage 700 nach der exothermen Reaktion zwischen Kohlendioxid 101 ($CO_2$) und Wasserstoff 103 ($H_2$) wie folgt erfolgen:

$$CO_2 + 3\ H_2 = CH_3OH + H_2O - 49{,}6\ kJ \text{ (Methanol-Wassergemisch, dampfförmig)}$$
$$\text{(Reaktion 2)}$$

Die entstehende Reaktionswärme von 49,6 kJ/mol = 1550 kJ pro kg Methanol = 0,43 kWh pro kg Methanol 108 wird aus dem entsprechenden Reaktor 10 abgeführt. Zu diesem Zweck umfasst der Reaktor 10 einen Fluidraum 14 (siehe z.B. Fig. 3), d.h. der Reaktor 10 ist von einem Reaktormantel umgeben und durch ein Fluid (vorzugsweise Wasser) gekühlt.

Typische Synthesebedingungen im Synthesereaktor 10 sind ca. 50 bis 80 bar und ca. 270 °C. Die Reaktionswärme kann z.B. an andere Anlagenelemente "übergeben" werden.

Die Methanol-Wasser-Synthese wird gemäss Erfindung unter Einsatz eines Katalysators 60 durchgeführt, um Reaktionstemperatur, Reaktionsdruck sowie Reaktionsdauer im Vergleich zu anderen Verfahren niedrig zu halten und um sicher zu stellen, dass als Reaktionsprodukt ein flüssiges Methanol-Wasser-Gemisch 108 entsteht, das als Kühlflüssigkeit geeignet ist.

Falls sich die Silicon-Fire Anlage 700 in der Nähe einer $CO_2$-Quelle (z.B. einem Schiff 60, wie in Fig. 4 gezeigt) befindet, kann auf eine Verflüssigung von $CO_2$ für den Transport verzichtet werden. Ansonsten ist es nach dem Stand der Technik relativ einfach, das $CO_2$ zu verflüssigen und auch über große Entfernungen zu einer Silicon-Fire Anlage 700 zu bringen. Bei einem Verzicht auf Verflüssigung und ggfs. auf Speicherung und Transport über längere Entfernung, ist das $CO_2$ unter Berücksichtigung von $CO_2$-Vermeidungs-Gutschriften in vielen Fällen kostenneutral verfügbar. Auch im Falle eines Transports sind die Kosten für das "Erwerben" des $CO_2$ relativ gering.

In Fig. 2 ist anhand des gestrichelten Pfeils 112 angedeutet, der von der Steuerung 110 ausgeht, dass die Steuerung 110 den Energiestrom E1 regelt. Der Pfeil 112 stellt eine Steuer- oder Signalleitung dar. Es sind auch andere mögliche Steuer- oder Signalleitungen 113, 114 dargestellt. Die Steuer- oder Signalleitung 113 regelt zum Beispiel die $CO_2$-Menge, die für die Reaktion 106 zur Verfügung steht. Wenn zum Beispiel weniger Wasserstoff 103 produziert wird, dann muss auch proportional weniger $CO_2$ zugeführt werden. Die optionale Steuer- oder Signalleitung 114 kann zum Beispiel die $H_2$-Menge regeln. Eine solche Regelung ist dann sinnvoll, wenn es einen Wasserstoff-Pufferspeicher gibt, dem man Wasserstoff 103 entnehmen kann, auch wenn im Moment kein Wasserstoff oder weniger Wasserstoff durch Elektrolyse 105 (oder durch den Einsatz elementaren Siliziums oder Metalls) produziert wird.

Untersuchungen haben ergeben, dass es besonders wirtschaftlich und umwelttechnisch sinnvoll ist, wenn die Silicon-Fire Anlage 100 oder 700 so ausgelegt oder gesteuert wird, dass zwischen 15 % und 40 % des Methanol-Wasser-Gemischs 108 aus regenerativer Energie erzeugt werden, während weiteres Methanol-Wasser-Gemisch für die Ergänzung zu 100 % aus anderen Kohlenwasserstoffen (z.B. aus Methangas) bereitgestellt wird.

Besonders bevorzugt ist eine Ausführungsform des Betriebskonzepts der Anlage 100 oder 700, die den Bezug kostengünstiger elektrischer Energie in den Schwachlastzeiten aus einem Verbundnetz 500 vorsieht (wie in Fig. 1 gezeigt).

Details einer besonders bevorzugten Ausführungsform eines Reaktors 10 zur Synthese des Methanol-Wasser-Gemischs 108 ist in den Fig. 3 gezeigt.

Die Ausführungen, die in der internationalen Patentanmeldung PCT/EP2010/064948, Anmeldedatum 6. Oktober 2010, zur Synthese von Methanol 108 gemacht werden, lassen sich auch auf die Synthese anderer flüssiger Kohlenwasserstoffe übertragen.

Das Methanol-Wasser-Gemisch 108 wird, wie bereits beschrieben, unter Einsatz eines Ausgangsstoffes AS, der $CO_2$-Gas 101 und Wasserstoffgas 103 enthält, synthetisiert. Der entsprechende Reaktor 10 umfasst ein Reaktorelement oder mehrere parallel zueinander angeordnete Reaktorelemente. Am Reaktor 10 gibt es mindestens einen Gaseintritt 21 für den Ausgangsstoff AS und einen Produktauslass 23, wie in Fig. 3 beispielhaft gezeigt.

Der Ausgangsstoff AS wird beim Durchlaufen respektive beim Hindurchpressen durch die Reaktorröhre(n) des Reaktors 10 sukzessive zu einem methanolhaltigen Gemisch 108 (alkoholhaltige Kühlflüssigkeit genannt) umgesetzt. Auf der Eingangsseite des Reaktors 10 liegt die Methanolkonzentration des Reaktionsfluids bei Null und die Konzentration des gasförmigen Ausgangsstoffes AS bei ca. 100 %. In Richtung Ausgangsseite des Reaktors 10 verschieben sich die entsprechenden Konzentrationen gegenläufig bis am Ausgang (am Produktauslass 23) ein methanolhaltiges Gemisch 108 mit einer vorgegebenen Methanolkonzentration (vorzugsweise ein Methanol-Wasser-Gemisch im Verhältnis 1:2) gebildet ist.

Der Reaktor 10 liefert als Rohmethanol ca. 64 Mass.-% (69,2 Vol.-%) Methanol und 36 Mass.-% (30,8 Vol.-%) Wasser.

Die Kühlflüssigkeit 108 sollte besonders vorzugsweise zwischen 5 und 50 Mass.-% Methanol und den Rest Wasser enthalten. Ganz besonders geeignet ist eine Kühlflüssigkeit 108 mit ca. 10 bis 15 Mass.-% Methanol, um die Kosten der Kühlflüssigkeit 108 und die Belastung der Wärmekraftmaschine 62 gering zu halten.

Der Reaktor 10 oder die Reaktorelemente des Reaktors 10 umfassen bei allen Ausführungsformen einen Katalysator für die Synthese des Methanol-Wasser-Gemischs 108.

Vorzugsweise kommt bei allen Ausführungsformen eine Steuerung des Reaktors 10 zum Einsatz, die anfangs beim "Hochfahren" des Reaktors 10 den Fluidraum 14 mit warmem Fluid beaufschlagt, um die Synthesereaktion in Gang zu setzen. Anschliessend wird vorzugsweise ein gekühltes Fluid zugeführt, um Reaktionswärme, die bei der exothermen Synthese entsteht, abzuführen und um so eine isotherme Umgebung zu schaffen.

Vorzugsweise ist der Fluidraum 14 bei allen Ausführungsformen so ausgelegt, dass mindestens die Reaktionsabschnitte des Reaktors 10, die mit dem Katalysator gefüllt sind, in der isothermen Umgebung liegen.

In Fig. 3 ist der Reaktor 10 von aussen gezeigt.

Vorzugsweise wird bei allen Ausführungsformen der Erfindung der Ausgangsstoff AS vorgewärmt und/oder mit erhöhtem Druck durch Zufuhrleitungen in den Reaktor 10 eingebracht. Der Druck und die Temperatur hängen von der Art des Katalysators ab. Vorzugsweise liegt die Temperatur im Bereich zwischen 100 und 350 °C. Der Druck liegt typischerweise zwischen 10 und 150 bar. Daher kann auch gesagt werden, dass der Ausgangsstoff AS vorzugsweise bei allen Ausführungsformen unter Vorgabe eines eingangsseitigen Drucks zwischen 10 und 150 bar durch den Reaktor 10 gepresst wird.

Der Reaktor 10 eignet sich speziell für die Synthese eines regenerativen Methanol-Wasser-Gemischs 108 aus Kohlendioxid $CO_2$ und Wasserstoff $H_2$, der über die (endotherme) Elektrolyse von Wasser mit regenerativer elektrischer Energie E1 gemäß Reaktion 1, wie bereits weiter oben erwähnt, erzeugt wird.

$$H_2O \ - \ 286{,}02 \ kJ/mol \ = \ H_2 \ + \ 0{,}5O_2 \ . \qquad\qquad (Reaktion \ 1)$$

Die exotherme Methanol-Wasser-Synthese (Reaktion 2, wie weiter oben bereits erwähnt) wird durch die Summenformel dargestellt:

$$CO_2 + 3\,H_2 \;=\; CH_3OH + H_2O - 49{,}6\ kJ\ (\text{Methanol gasförmig}). \qquad (\text{Reaktion 2})$$

In der Praxis wird vorzugsweise die zur Verfügung stehende regenerative elektrische Energie E1 zur Erzeugung des "regenerativen" Methanol-Wasser-Gemischs 108 gemäß den Reaktionen 1 und 2 maximal ausgenutzt, und der optionale Anteil von "fossil" erzeugtem Methanols wird nach wirtschaftlichen und ökologischen Zielsetzungen und Randbedingungen bis zum möglichen Maximalwert eingestellt, z.B. nach gewünschter spezifischer $CO_2$-Emission des "Gesamt"-Methanol-Wasser-Gemischs bei Verbrennung oder nach dem momentanen Preis und der Verfügbarkeit des Erdgases oder nach der zu erzeugenden "Gesamt"-Methanolmenge oder nach den Preisen der regenerativen und fossilen Methanolanteile.

Es muss betont werden, dass in allen Ausführungsformen natürlich auch andere Syntheseverfahren und andere Reaktoren 10 oder Anlagen eingesetzt werden können und dass die Synthese mit regenerativer Energie und/oder mit regenerativem Ausgangsstoff AS betrieben werden kann. Bevorzugt ist der Einsatz regenerativer Energie und regenerativer Ausgangsstoffe AS.

Besonders vorteilhaft ist, dass das "regenerative" Methanol-Wasser-Gemisch 108 und das "fossile" Methanol-Wasser-Gemisch, falls vorhanden, in verschiedenen Reaktoren getrennt anfallen können und entweder getrennt abgegeben werden können oder nach dem Anfall und ggf. Zwischenlagerung in beliebigen Anteilen gemischt werden können, so dass z.B. die Silicon-Fire Anlage 100 rein "regeneratives" Methanol-Wasser-Gemisch 108 und rein "fossiles" Methanol-Wasser-Gemisch liefern kann, aber auch beliebige Mischungen von beiden, um z.B. als regenerative Kühlflüssigkeit für die Ansaug- oder Zwischenkühlung mit zulässigem fossilen Anteil oder zulässiger spezifischer $CO_2$-Emission vermarktet werden zu können.

Besonders vorteilhaft ist der Einsatz der Erfindung im Zusammenhang mit einem Verfahren zur Methanol-Wasser-Synthese, das bei niedrigen Drücken zwischen 10 und 150 bar (vorzugsweise bei ca. 80 bar) arbeitet.

Das Prinzip der Erfindung lässt sich auch auf Grossanlagen übertragen, eignet sich aber besonders für kleine Lokalanlagen, die zum Beispiel in einem Seefracht-Container installiert sind.

Gemäß Erfindung dient $CO_2$ 101 als Ausgangsstoff und Kohlenstofflieferant für die Methanol-Wasser-Synthese im Reaktor 10. Als $CO_2$-Quellen dienen vorzugsweise: Steam-Reforming-Anlagen, $CO_2$-Abscheidungsanlagen für Roherdgas, Kalkbrennöfen, Kalzinieranlagen für Soda, Fermentationsanlagen für Bioethanol, Meerwasserentsalzungsanlagen, große Feuerungsanlagen für fossile Brennstoffe (z.B. Kraftwerksfeuerungen), Verbrennungskraftmaschinen von Fahrzeugen 60 und andere Wärmekraftmaschinen 62 oder Verbrennungsprozesse, die relativ große Mengen an $CO_2$ emittieren.

Je nach Synthesereaktion können z.B. kupfer-basierte Katalysatoren (z.B. CuO-Katalysatoren), oder Zinkoxid-Katalysatoren (z.B. ZnO-Katalysatoren), oder Chromoxid-Zinkoxid-Katalysatoren eingesetzt werden. Auch alle anderen bekannten Katalysatoren eignen sich für den Einsatz in einem Reaktor 10. Besonders geeignet sind Festbett-Katalysatoren oder Flüssigbett-Katalysatoren. Der Katalysator kann auch einen geeigneten Träger (z.B. Karbon, Silikat, Aluminium (z.B. $Al_2O_3$) oder Keramik umfassen. Statt der erwähnten "metallischen" Katalysatoren kann auch ein organischer Katalysator eingesetzt werden.

Der Katalysator hat vorzugsweise bei allen Ausführungsformen eine Korn-, Kugel- oder Teilchengrösse zwischen 1 und 10 mm. Besonders bevorzugt ist eine Korn-, Kugel- oder Teilchengrösse zwischen 3 und 8 mm.

Kohlendioxid als Kohlenstofflieferant kann gemäß Erfindung auch aus dem Roherdgas entnommen werden, das je nach Erdgasquelle mehr als 10 % Kohlenstoffdioxid-Anteil aufweisen kann. Nach dem Fördern des Roherdgases wird bereits heute typischerweise eine Gastrennung (mittels Gaswäschetechnologie oder einer anderen Gastrenntechnologie) durchgeführt, um das $CO_2$ vom eigentlichen Erdgas zu trennen. Meist wird dieses $CO_2$ in die Atmosphäre emittiert. Gemäß Erfindung kann das $CO_2$, das in weitgehend reiner Form vorliegt, als Kohlenstofflieferant 101 eingesetzt werden.

Gemäss Erfindung wird das regenerative oder das regenerative und fossile Methanol-Wasser-Gemisch 108 als Kühlflüssigkeit zum Ansaug- oder Zwischenkühlen eingesetzt, wie z.B. in Fig. 5 angedeutet.

Das Methanol-Wasser-Gemisch 108 eignet sich besonders zur Nutzung als aktive Kühlflüssigkeit in einem Nebenaggregat 65 (Verdichter) einer Verbrennungskraftmaschine 62, z.B. in einem Zwischenkühlaggregat 65 eines Fahrzeugs 60. Der Begriff "aktive Kühlflüssigkeit" soll andeuten, dass der Alkoholanteil in der Verbrennungskraftmaschine 62 mit verbrennt.

Der Verdichter 65 ist als ein Bestandteil der Verbrennungskraftmaschine 62 zu betrachten.

Das Verfahren zum Bereitstellen und Verbrauchen einer speicherbaren und transportablen alkoholhaltigen Kühlflüssigkeit 108 umfasst somit folgende Schritte:

- Bereitstellen (Schritt 104 in Fig. 1, 2) eines Gases mit Kohlenstoffdioxidanteil ($CO_2$) 101 als Kohlenstofflieferant. Das Bereitstellen kann z.B. durch direkte oder indirekte Entnahme über eine Verbindung 52 (siehe Fig. 4) erfolgen.
- Bereitstellen eines Wasserstoffanteils 103 ($H_2$). Der Wasserstoffanteil 103 wird vorzugsweise, wie beschrieben, elektrolytisch aus Wasser ($H_2O$) erzeugt.
- Bereitstellen eines Ausgangsstoffes AS, der den Kohlenstoffdioxidanteil 101 und den Wasserstoffanteil 103 umfasst.
- Einbringen des Ausgangsstoffes AS in einen Reaktor 10 (z.B. wie in Fig. 3 gezeigt).
- Durchlaufen oder Hindurchpressen des Ausgangsstoffs AS durch eine Reaktionsstrecke des Reaktors 10, die mindestens teilweise mit einem Katalysator bestückt ist. In diesem Schritt wird in einem katalytischen Syntheseverfahren die alkoholhaltige Kühlflüssigkeit synthetisiert.
- Bereitstellen der alkoholhaltigen Kühlflüssigkeit 108 an einem ausgangsseitigen Ende 23 des Reaktors 10, wobei es sich bei der alkoholhaltigen Kühlflüssigkeit 108 um ein Gemisch aus einem Alkoholanteil (z.B. Methanol und/ oder Ethanol) und einem Wasseranteil handelt.
- Einsetzen dieser alkoholhaltigen Kühlflüssigkeit 108 in einer Verbrennungskraftmaschine 62 (z.B. in einem Verbrennungsmotor), die mit Luft oder Kraftstoff-Luft-Gemisch beschickt wird, wobei die alkoholhaltige Kühlflüssigkeit 108 eingesetzt wird, um durch Verdampfen der alkoholhaltigen Kühlflüssigkeit 108 die Luft oder das Kraftstoff-Luft-Gemisch zu kühlen.

Bei dem Kraftstoff kann es sich um einen Dieselkraftstoff, einen dieselähnlichen Kraftstoff (z.B. Bio-Diesel), einen Otto-Kraftstoff, ein Kohlenwasserstoffgas oder einen Spezialkraftstoff (z.B. Kerosin) handeln.

Die Rückkühlung des Gases, respektive der Ladeluft der Verbrennungskraftmaschine 62, mit entsprechenden Leistungs- und Wirkungsgradgewinnen und ohne Druckverlust kann durch das Einbringen/Einspritzen der Kühlflüssigkeit 108 in die Luft oder das Kraftstoff-Luft-Gemisch, wobei die Kühlflüssigkeit 108 verdampft und entsprechend ihrer Verdampfungswärme die Luft oder das Kraftstoff-Luft-Gemisch abkühlt.

Da die Kühlflüssigkeit 108 brennbar ist, nimmt sie am nachfolgenden Verbrennungsvorgang in der Wärmeumwandlungsanlage 64 unter entsprechender Abgabe mechanischer Leistung und/oder Wärme teil. Die Kühlflüssigkeit wird daher hier auch als aktive Kühlflüssigkeit beizeichnet.

Es ist auch möglich, bei Diesel-Motoren einen entsprechenden Teil des Primärkraftstoffs in Form eines sonst wegen ungenügender Zündeigenschaften (z.B. zu niedrige Cetan-Zahl) nicht für Diesel-Motoren geeigneten flüssigen Energieträgers (wie Ethanol oder Methanol) zuzuführen. Methanol-Wasser-Gemische 108 sind wegen ihrer relativ hohen Verdampfungswärme für die genannte Form der Ansaug- oder Zwischenkühlung besonders geeignet.

Am Beispiel eines LKW-Diesel-Motors sollen die Relationen beim Einspritzen von Methanol zum Rückkühlen der Ladeluft dargestellt werden.

Angenommene Parameter: Motordaten des Viertakt-Turbodiesel-Motors bei Nennleistung:

| | |
|---|---|
| Leistung: | 220 kW |
| Ladedruck (absolut): | 2,4 bar |
| Außenlufttemperatur: | 21,0 °C |
| Lufttemperatur vor Verdichter: | 31,0 °C |
| Lufttemperatur nach Verdichter und vor Rückkühlung: | 117,0 °C |
| Lufttemperatur nach Rückkühlung: | 40,0 °C |
| Motor-Wirkungsgrad: | 43 % |
| Motor-Diesel-Kraftstoffverbrauch (Dieselkraftstoff-Massenstrom): | 0,0119 kg/s |
| stöchiometrisches Luft-Kraftstoff-Verhältnis: | 2 |
| Luft-Kraftstoff-Massenverhältnis: | 14,9 |
| Motor-Luftverbrauch (Luft-Massenstrom): | 0,177 kg/s |
| isobare spezifische Wärmekapazität von Luft: | 1,0 kJ/(kg.K) |
| Heizwert von Dieselkraftstoff: | 43,0 MJ/kg |
| Heizwert von Methanol: | 19,9 MJ/kg |
| spezifische Verdampfungswärme von Methanol (bei 65 °C): | 1099 kJ/kg |

Für die Abkühlung der aufgeladenen Luft von 117 °C auf 40 °C (um 77 K) gilt folgende Energiebilanz:

Massenstrom Luft x Temperaturdifferenz Luft x isobare spezifische Wärmekapazität Luft = Massenstrom Methanol x spezifische Verdampfungswärme Methanol.

Daraus folgt: Massenstrom Methanol = 0,0124 kg/s entsprechend einem Heizwertstrom von 0,0124 kg/s x 19,9 MJ/kg = 0,247 MJ/s, der den entsprechenden Dieselkraftstoff-Heizwertstrom ersetzt.

Der ursprüngliche Dieselkraftstoff-Heizwertstrom ist 0,0119 kg/s x 43 MJ/kg = 0,512 MJ/s, davon werden 0,247 MJ/s durch Methanol ersetzt; verbleiben als Dieselkraftstoff-Heizwertstrom 0,265 MJ/s bzw. als Dieselkraftstoff-Massenstrom 0,00616 kg/s.

Damit liefert nach der oben beispielhaft beschriebenen Methanoleinspritzung der Dieselkraftstoff 52 % des gesamten erforderlichen Heizwert-Massenstromes und das Methanol 48 %.

Der Verbrennungskraftmaschine 62 würde damit bei Rückkühlung (Zwischenkühlung) der Ladeluft um 77 K mit Methanol etwa die Hälfte der Kraftstoffenergie über Dieselkraftstoff als Primärkraftstoff zugeführt, der - wie beim Diesel-Prozess üblich - entsprechend dem "Einspritzgesetz" im Verbrennungsraum während des Arbeitstaktes (bei Vergrößerung des Verbrennungsraumes durch die Kolbenbewegung) bei etwa konstantem Druck verbrennt, während die andere Hälfte der Kraftstoffenergie über Methanol zugeführt wird, das - im Zwischenkühler 65 vorgemischt mit der Verbrennungsluft - im Moment des ersten Entflammens von eingespritztem Dieselkraftstoff entzündet wird und explosionsartig verbrennt mit entsprechend steilem Druckanstieg im Verbrennungsraum 62 der Verbrennungskraftmaschine und mechanischer und thermischer Belastung der Motorbauteile.

Ein nicht entsprechend ausgelegter Diesel-Motor wird diesem "Mischbetrieb" zwischen Otto- und Diesel-Prozess auf Dauer nicht stand halten. Deshalb umfasst gemäss Erfindung die Flüssigkeit 108 außer Methanol zur Hälfte bis zu zwei Dritteln Wasser, d.h. es kommt ein Gemisch 108 zum Einsatz, das in der beschriebenen Art und Weise hergestellt wurde.

Bei der Einspritzung/Eindüsung/Beimengung des Methanol-WasserGemisches 108 zur Ladeluftkühlung kann bei Verwendung der beschriebenen Verfahren der von EU- und nationalen Vorschriften geforderte regenerative Energieanteil von 5,75 % im Fahrzeugkraftstoff bei aufgeladenen Otto- und Diesel-Motoren ohne Zumischung von Bioethanol oder Biodiesel zum Kraftstoff erreicht und überschritten werden.

Der oben genannte regenerative Energieanteil von 5,75 % kann auch erreicht werden bei aufgeladenen Standard-Otto- und -Diesel-Motoren mit Ladeluftkühler durch zusätzliche Einspritzung eines Methanol-Wasser-Gemischs 108 nach dem Ladeluftkühler und dadurch zusätzliche Abkühlung der Ladeluft.

5,75 % Energieanteil im Kraftstoff des oben betrachteten Diesel-Motors mit einem gesamten Kraftstoff-Heizwertstrom von 0,512 MJ/s entsprechen 0,0256 MJ/s oder einem Methanolmassenstrom von 0,0256 MJ/s :

$$19,9 \ MJ/kg = 0,00129 \ kg/s = 1,29 \ g/s = 4,63 \ kg/h.$$

Die zusätzliche Abkühlung der Ladeluft nach dem Ladeluftkühler beträgt durch die Methanoleinspritzung 8,0 K, was zu einer merklichen Erhöhung von Leistung und Wirkungsgrad der Verbrennungskraftmaschine 62 führt.

Die Einspritzung eines Methanol-Wasser-Gemischs 108 zur Ladeluftkühlung eröffnet für die weit verbreiteten aufgeladenen Otto- und Diesel-Verbrennungskraftmaschinen eine leicht nachrüstbare Möglichkeit, den zur Zeit geforderten 5,75 %igen regenerativen Energieanteil in Fahrzeugkraftstoffen ohne Zumischung von Bioethanol oder Biodiesel zum Primärkraftstoff weit zu übertreffen bei zusätzlich dadurch erzielter nennenswerter Erhöhung von Leistung und Wirkungsgrad der Verbrennungskraftmaschinen 62.

Im Folgenden werden weitere bevorzugte Aspekte eines erfindungsgemäßen Verfahrens beschrieben. Es wird dabei auf die schematische Fig. 4 verwiesen.

Das Gemisch 108, das am ausgangsseitigen Ende 23 des Reaktors 10 bereit gestellt wird, umfasst vorzugsweise Methanol als Alkoholanteil. Das Mischungsverhältnis von Methanol zu Wasser beträgt vorzugsweise bei allen Ausführungsformen zwischen 5 Mass.-% zu 95 Mass.-% und 50 Mass.-% zu 50 Mass.-%. Damit werden bei der Zwischenkühlung besonders gute Ergebnisse erzielt.

Das Mischungsverhältnis von Methanol zu Wasser umfasst vorzugsweise bei allen Ausführungsformen weniger als 30 Mass.-% Methanol und mehr als 70 Mass.-% Wasser.

Ganz besonders geeignet ist ein Mischungsverhältnis von Methanol zu Wasser zwischen 10 und 15 Mass.-% Methanol und zwischen 90 und 85 Mass.-% Wasser.

Falls der Wasseranteil des Gemischs 108, das eingesetzt wird, höher sein muss als der Wasseranteil des Gemischs 108, das am ausgangsseitigen Ende 23 des Reaktors 10 in einem integrierten Verfahren bereit gestellt wird, kann der Wasseranteil des Gemischs 108 durch Zuführen von zusätzlichem Wasser nachträglich erhöht werden.

Da die Kühlflüssigkeit 108 einen Alkoholanteil umfasst, nimmt dieser Alkoholanteil an der Verbrennung in der Verbrennungskraftmaschine 62 teil, wenn die Kühlflüssigkeit 108 in die Ladeluft oder das Kraftstoff-Luft-Gemisch der Verbrennungskraftmaschine 62 eingebracht wird. Dass heißt, der Alkoholanteil der Kühlflüssigkeit 108 nimmt zusammen mit dem Primärkraftstoff an der Verbrennung in der Verbrennungskraftmaschine 62 teil.

Vorzugsweise wird bei allen Ausführungsformen die Menge der Kühlflüssigkeit 108, die eingesetzt wird, bedarfsabhängig geregelt.

Vorzugsweise wird die Kühlflüssigkeit 108 bei allen Ausführungsformen in einem Zwischenschritt in einen (Zweit-)Tank 61 eines Fahrzeugs 60 gespeichert und bei Bedarf aus dem Tank 61 des Fahrzeugs 60 entnommen und der Verbrennungskraftmaschine 62 zur Ansaug- oder Zwischenkühlung zugeführt, wie in Fig. 4 angedeutet.

Vorzugsweise kommt das Verfahren zur Ansaug- oder Zwischenkühlung bei einem Schiff 60, einem Strassen- oder Geländefahrzeug (z.B. einem Bus, einem LKW oder einem Sonderfahrzeug), oder bei einem Flugzeug zum Einsatz.

Die Erfindung eignet sich besonders zum Einsatz in einem Hafen, wie in Fig. 4 schematisch angedeutet. Ein Schiff 60, das in dem Hafen angedockt hat, lässt typischerweise seine Generatoren und andere Aggregate laufen, um das Schiff 60 mit der erforderlichen Energie zu versorgen. Diese Vorgehensweise führt zu sehr starken Emissionen. Gemäss Erfindung kann ein im Hafen vor Anker liegendes Schiff 60 mit einer Fangvorrichtung zum Einfangen von $CO_2$ ausgestattet sein. Die Entnahme des $CO_2$ ist in Fig. 4 schematisch durch die Verbindung 52 veranschaulicht. Der Wasserstoff kann z.B. über eine Elektrolyse 105 aus Wasser hergestellt werden, wie in Fig. 4 gezeigt. Das Gasgemisch (Ausgangsstoff AS) wird in einem Reaktor (z.B. in einem Reaktor 10 nach Fig. 3) auf katalytischen Wege zu einem Methanol-Wasser-Gemisch 108 synthetisiert. Dieses Gemisch 108 kann in einen (Zweit-)Tank 61 des Schiffs 60 gefüllt werden. Das Betanken ist durch eine Tanksäule 50 und eine Leitung 51 versinnbildlicht. Die Verbrennungskraftanlage 62 (z.B. ein Dieselmotor des Schiffs 60) weist Mittel auf, um die Kühlflüssigkeit 108 in den Luftstrom oder das Kraftstoff-Luft-Gemisch einzubringen. Durch diese Massnahme können die Emissionen und der Verbrauch des Schiffs 60 reduziert werden.

Die Zwischenkühlung, wie beschrieben, kann auch während der Verdichtung bei einer Gasturbinenanlage eingesetzt werden. Eine solche Gasturbinenanlage umfasst in diesem Fall mindestens einen ersten Verdichter 65, eine Brennkammer und eine Turbine. Die Brennkammer bildet zusammen mit der Turbine eine Wärmeumwandlungsanlage 64. Der erste Verdichter 65 kann mit einer Ansaugluft- oder/und einer Zwischenkühlung ausgestattet sein, die mit dem Alkohol-Wasser-Gemisch 108, wie beschrieben, beschickt wird. Das Alkohol-Wasser-Gemisch 108 kann aus einem Zweittank 61 zugeführt werden.

Die Vorteile der Erfindung sind unter anderem darin zu sehen, dass durch die Ansaug- und/oder Zwischenkühlung der Gesamtwirkungsgrad und die Leistung der Verbrennungskraftmaschine 62 erhöht werden können. Durch den erhöhten Wirkungsgrad wird die Umweltbilanz der Verbrennungskraftmaschine 62 verbessert, da im Vergleich zur gewonnenen Leistung weniger Schadstoffe produziert werden.

Es ist ein Vorteil der Erfindung, dass die entsprechende Ansaug- und Zwischenkühlung nachgerüstet werden können.

Das erfindungsgemässe System zur Ansaug- und Zwischenkühlung umfasst vorzugsweise bei allen Ausführungsformen einen Zweittank 61, eine Vorrichtung zum Einspritzen oder Einbringen der Kühlflüssigkeit 108, und Mittel zum Einbinden der erwähnten Komponenten in eine Steuerung oder Regelung der Verbrennungskraftanlage 62.

Das erfindungsgemässe System zur Ansaug- und Zwischenkühlung ist somit ein Wärmeübertragungssystem, das im Ansaugtrakt oder im Verdichter 65 einer Verbrennungskraftmaschine 62 die Temperatur des der Maschine 62 zugeführten Mediums (Luft oder Kraftstoff-Luft-Gemisch) durch Einspritzen oder Einbringen der Kühlflüssigkeit 108 verringert.

Die Kühlflüssigkeit 108 kann in einem Turbomotor, Kompressormotor oder Saugmotor zur Ansaug- bzw. Zwischenkühlung eingesetzt werden. Der Alkoholanteil der Kühlflüssigkeit 108 dient als zusätzlicher Kraftstoff und Sauerstoffträger, was bei Diesel-Motoren ein besonderer Vorteil ist, für die Alkohole als Primärkraftstoff wegen zu geringer Cetan-Zahl nicht geeignet sind.

Das Verfahren zeichnet sich dadurch aus, dass die Kühlflüssigkeit 108 in einem oder mehreren Reaktoren 10 an Bord eines Fahrzeugs 60 hergestellt und in einem Tank 61 des Fahrzeugs 60 zwischengespeichert werden kann. Sämtliche Ausführungsformen der Erfindung lassen sich in einem Fahrzeug 60 anwenden. Bei der Verwendung des Verfahrens im Fahrzeug 60 werden vorzugsweise Mikroreaktoren 10 und/oder Mikrokatalysatoren zur Synthese der Kühlflüssigkeit 108 eingesetzt. Diese Mikroreaktoren 10 und/oder Mikrokatalysatoren sind vorzugsweise mit der Verbrennungskraftmaschine 62 (z.B. mit dem Schiffsdiesel) wärmetechnisch so gekoppelt, das Abwärme, so erforderlich, von der Verbrennungskraftmaschine 62 an die Mikroreaktoren 10 und/oder Mikrokatalysatoren übergeben wird. Gemäss Erfindung können in einem Fahrzeug 60 sogenannte chemische Kleinkreisläufe realisiert werden, die $CO_2$ aus Abgasen mit lokal vorhandener Energie (Abwärme, Wind- oder Solarstrom, Strömungsenergie aus Wasserströmung) einsetzen, um die Kühlflüssigkeit 108 bereit zu stellen.

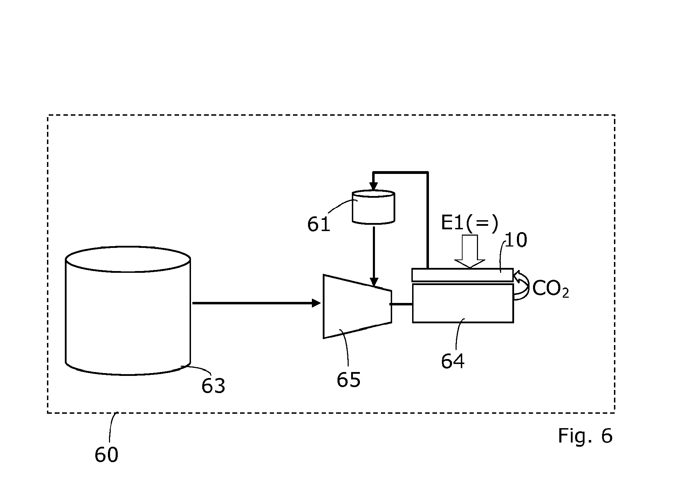

Fig. 6 zeigt eine schematische Ansicht einer Vorrichtung, die z.B. Teil eines Fahrzeugs 60 ist und die im gezeigten Beispiel einen Verdichter 65, eine Wärmeumwandlungsanlage 64 und einen Mikroreaktor 10 umfasst. Primärkraftstoff (z.B. Schiffsdiesel-Kraftstoff) wird aus einem Tank 63 der Wärmeumwandlungsanlage 64 zugeführt. $CO_2$-Abgase (hier als Pfeil gezeigt) werden von der Wärmeumwandlungsanlage 64 in einen Mikroreaktor 10 eingebracht. Dort wird Wasserstoffgas auf katalytischem Wege mit dem $CO_2$ zu der Kühlflüssigkeit 108 umgesetzt. Das Wasserstoffgas kann durch Zuführen von Gleichstromenergie E1 erzeugt werden. Das Gemisch 108 gelangt in einen Tank 61. Von dort wird die Kühlflüssigkeit 108 entnommen und z.B. vor bzw. in einem Verdichter 65 zur Ansaug- bzw. Zwischenkühlung, wie beschrieben, zum Einsatz gebracht.

Die Ansaugkühlung und die Zwischenkühlung werden hier in vorteilhafter Weise durch das Einbringen einer Kühlflüssigkeit (Alkohol-Wasser-Gemisch 108) in die Luft oder das Kraftstoff-Luft-Gemisch mit deren anschließender Verdampfung erzielt, wodurch die Luft oder das Kraftstoff-Luft-Gemisch durch Entzug der Verdampfungswärme der Kühlflüssigkeit abgekühlt wird. Diese Art der Kühlung ist ohne wesentlichen Bauaufwand zu realisieren und führt zu praktisch keinem Druckverlust. Sie hat die Zusatzvorteile, dass die Abkühlung bis weit unter die Umgebungstemperatur möglich ist und dass brennbare Bestandteile der Kühlflüssigkeit (Alkohol-Wasser-Gemisch 108) sich an der anschließenden Verbrennung beteiligen und dadurch auch den entsprechenden Anteil an der Wärmezufuhr zur Luft oder zum Kraftstoff-Luft-Gemisch (Arbeitsmedium) haben. Dadurch kann ein Alkohol-Wasser-Gemisch 108 ein Teil des Kraftstoffes z.B. von Diesel-Motoren sein, obwohl eine solche Kühlflüssigkeit 108 sonst für Diesel-Motoren wegen mangelnder Zündwilligkeit (wegen der zu geringen Cetan-Zahl) nicht geeignet ist.

Durch die Erfindung ergeben sich neben ökologischen Effekten auch nachhaltige Kostenvorteile.

Besonders interessant ist das Verfahren für umweltsensible Anwendungssegmente, wie zum Beispiel öffentlichen Personennahverkehr, Logistikunternehmen, Entsorgungsunternehmen, land- und forstwirtschaftliche Maschinen, Flughäfen oder Baumaschinen im Untertagebau, und andere Gebiete mit hohen Nachhaltigkeitsanforderungen.

Ein entsprechendes Verdichteraggregat 65 kann bei allen Ausführungsformen mit einer Gasführung oder Gasleitung ausgestattet sein, um die Kühlflüssigkeit 108 in den Luftstrom oder in das Kraftstoff-Luft-Gemisch einzubringen.

Die Ansaug- oder Zwischenkühlung mit einem Methanol-Wasser-Gemisch 108 anstelle von Wasser ist besonders vorteilhaft, weil sie wesentlich weniger abhängig vom Feuchtigkeitsgehalt der Ansaugluft ist.

Das hier beschriebene Verfahren mit einem Alkohol-Wasser-Gemisch 108 als Kühlflüssigkeit eignet sich nur bedingt für Gasturbinen, bei denen ein Teil der Verdichterluft als Kühlluft für Brennkammer und Turbinenteil verwendet wird. Die Entzündungsmöglichkeit des Alkohols in der Gasströmung und deren Folgen müssen berücksichtigt werden.

Bezugszeichen:

| | |
|---|---|
| Reaktor | 10 |
| Fluidraum | 14 |
| Fluidzufuhr | 16 |
| Fluidabfuhr | 17 |
| | |
| Gaseintritt | 21 |
| Produktauslass | 23 |
| | |
| Tankstelle | 50 |
| Betankungsvorgang | 51 |
| CO2-Entnahme | 52 |
| | |
| Fahrzeug (z.B. Schiff) | 60 |
| 2. Tank | 61 |
| Verbrennungskraftmaschine | 62 |
| 1. Tank (Primärkraftstofftank) | 63 |
| Wärmeumwandlungsanlage | 64 |
| Verdichter (optional) | 65 |
| | |
| Silicon-Fire Anlage | 100 |
| Kohlenstoffdioxid | 101 |

(fortgesetzt)

| Wasser | 102 |
|---|---|
| Wasserstoff | 103 |
| Bereitstellen von Kohlenstoffdioxid | 104 |
| Durchführen einer Elektrolyse | 105 |
| Abgeben/Bereitstellen von Methanol | 107 |
| transportable Kühlflüssigkeit (Gemisch) | 108 |
| (Anlagen-)Steuerung | 110 |
| Parameterspeicher | 111 |
| Steuer- oder Signalleitungen | 112, 113, 114, 115 |
| Solarthermieanlage | 300 |
| Umwandlung von Wärme in Gleichstrom | 301 |
| Solaranlage (Photovoltaikanlage) | 400 |
| Verbundnetz | 500 |
| Umwandlung von Wechselspannung in Gleichstrom (Energieversorgungsanlage) | 501 |
| | |
| Silicon-Fire Anlage | 700 |
| | |
| Ausgangsstoff | AS |
| Gleichstromenergie | E1 |
| Inputgrößen | I1, I2, usw. |
| Primärenergie | P1, P2 |

## Patentansprüche

1. Verfahren zum Bereitstellen und Einsetzen einer speicherbaren und transportablen, alkoholhaltigen Kühlflüssigkeit (108) mit den folgenden Schritten:

   - Bereitstellen (52; 104) eines Kohlenstoffdioxidgases (101) als Kohlenstofflieferant,
   - Bereitstellen eines Wasserstoffgases (103),
   - Bereitstellen eines gasförmigen Ausgangsstoffes (AS), der das Kohlenstoffdioxidgas (101) und Wasserstoffgas (103) umfasst,
   - Einbringen des Ausgangsstoffes (AS) in einen Reaktor (10),
   - Durchlaufen des Ausgangsstoffes (AS) durch eine Reaktionstrecke des Reaktors (10), die mindestens teilweise mit einem Katalysator bestückt ist, um auf katalysator-synthetischem Weg die Kühlflüssigkeit (108) zu synthetisieren,
   - Bereitstellen der Kühlflüssigkeit (108) an einem ausgangsseitigen Ende (23) des Reaktors (10), wobei es sich bei der Kühlflüssigkeit (108) um ein Gemisch (108) aus Alkohol und Wasser handelt,
   - Einsetzen der Kühlflüssigkeit (108) vor oder in einer Verbrennungskraftmaschine (62), die mit einem Kraftstoff beschickt wird, wobei die Kühlflüssigkeit (108) eingesetzt wird, um durch Verdampfen der Kühlflüssigkeit (108) Ansaugluft, Verdichterluft, Ladeluft oder/und ein Kraftstoff-Luft-Gemisch zu kühlen, welche der Verbrennungskraftmaschine (62) zugeführt wird.

2. Verfahren nach Anspruch 1, **dadurch gekennzeichnet, dass** die Kühlflüssigkeit (108), die am ausgangsseitigen Ende (23) des Reaktors (10) bereit gestellt wird, Methanol als Alkohol umfasst, und zwischen 5 und 50 Mass.- % Methanol und den Rest Wasser enthält.

**3.** Verfahren nach Anspruch 1, **dadurch gekennzeichnet, dass** die Kühlflüssigkeit (108), die am ausgangsseitigen Ende (23) des Reaktors (10) bereit gestellt wird, Methanol als Alkohol umfasst, und dass die Kühlflüssigkeit (108) weniger als 30 Mass.-% Methanol und mehr als 70 Mass.-% Wasser umfasst.

**4.** Verfahren nach Anspruch 2 oder 3, **dadurch gekennzeichnet, dass** das Wasser der Kühlflüssigkeit (108), die in der Verbrennungskraftmaschine (62) eingesetzt wird, einen Wasseranteil hat, der höher ist als der Wasseranteil der Kühlflüssigkeit (108), die am ausgangsseitigen Ende (23) des Reaktors (10) bereit gestellt wird, wobei der Wasseranteil durch Zuführen von zusätzlichem Wasser erhöht wird.

**5.** Verfahren nach Anspruch 1, 2 oder 3, **dadurch gekennzeichnet, dass** der Alkohol der Kühlflüssigkeit (108) an einer inneren Verbrennung in der Verbrennungskraftmaschine (62) teil nimmt.

**6.** Verfahren nach Anspruch 1, 2 oder 3, **dadurch gekennzeichnet, dass** der Alkohol der Kühlflüssigkeit (108) zusammen mit Kraftstoff an einer inneren Verbrennung in der Verbrennungskraftmaschine (62) teil nimmt.

**7.** Verfahren nach einem der vorhergehenden Ansprüche, **dadurch gekennzeichnet, dass** die Kühlflüssigkeit (108) zum Zwecke einer Ansaug- oder/und Zwischenkühlung eingesetzt wird.

**8.** Verfahren nach einem der vorhergehenden Ansprüche 1-6, **dadurch gekennzeichnet, dass** die Kühlflüssigkeit (108) in einem Verdichter (65) der Verbrennungskraftmaschine (62) zur Kühlung eingesetzt wird.

**9.** Verfahren nach einem der vorhergehenden Ansprüche 1-8, **dadurch gekennzeichnet, dass**

- die Menge der Kühlflüssigkeit (108), die eingesetzt wird, bedarfsabhängig geregelt wird, und/oder
- die Kühlflüssigkeit (108) mindestens anteilsmäßig $CO_2$-neutral hergestellt wurde.

**10.** Verfahren nach einem der vorhergehenden Ansprüche, **dadurch gekennzeichnet, dass** das Bereitstellen des Wasserstoffgases (103) geschieht, indem entweder

- eine Wasserelektrolyse (105) ausgeführt wird, bei der direkt aus Wasser oder einer wässerigen Lösung ($H_2O$; 102) das Wasserstoffgas (103) erzeugt wird, oder
- ein Energieträger, der ein reduziertes Metall, vorzugsweise elementares Silizium, enthält, einer Oxidationsreaktion unterzogen wird, um das Wasserstoffgas (103) aus einer wässerigen Lösung zu erzeugen.

**11.** Verfahren nach einem der vorhergehenden Ansprüche, **dadurch gekennzeichnet, dass** die Kühlflüssigkeit (108) in einem Zwischenschritt in einem Tank (61) eines Fahrzeugs (60) gespeichert und bei Bedarf aus dem Tank (61) des Fahrzeugs (60) entnommen und eingesetzt wird.

**12.** Verfahren nach einem der vorhergehenden Ansprüche, **dadurch gekennzeichnet, dass** das der Kohlenstoffdioxidanteil (101) aus den Abgasen eines Verbrennungsprozesses entnommen wird.

**13.** Verfahren nach Anspruch 11, **dadurch gekennzeichnet, dass** es sich bei dem Fahrzeug (60) um

- ein Schiff,
- ein Strassen- oder Geländefahrzeug, oder
- um ein Flugzeug handelt.

**14.** Verfahren nach Anspruch 1, **dadurch gekennzeichnet, dass** die Kühlflüssigkeit (108) in einem oder mehreren Reaktoren (10) an Bord eines Fahrzeugs (60) hergestellt und in einem Tank (61) des Fahrzeugs (60) zwischengespeichert wird.

**15.** Verwendung einer Kühlflüssigkeit (108), die Alkohol und Wasser umfasst, wobei der Alkohol unter Einsatz von Kohlenstoffdioxidgas (101) und Wasserstoffgas (103) synthetisiert und zusammen mit dem Wasser als Kühlflüssigkeit (108) in einem integrierten Prozess bereit gestellt wurde, um in einer Verbrennungskraftmaschine (62), die mit einem Kraftstoff beschickt wird, durch Verdampfen der Kühlflüssigkeit (108) Ansaugluft, Verdichterluft oder/und Kraftstoff-Luft-Gemisch der Verbrennungskraftmaschine (62) zu kühlen.

**16.** Verwendung nach Anspruch 15, **dadurch gekennzeichnet, dass** die Kühlflüssigkeit (108) in einem Tank (61) eines Fahrzeugs (60) gespeichert und bei Bedarf aus dem Tank (61) entnommen und eingesetzt wird.

17. Verwendung nach Anspruch 15 oder 16, **dadurch gekennzeichnet, dass** die Kühlflüssigkeit (108) in einem Turbomotor, Kompressormotor oder Saugmotor zur Ansaug- und/oder Zwischenkühlung eingesetzt wird.

Fig. 1

700

300 →

← 400

301

E1(=)

| H₂O 102 | | H₂ 103 |

105

| CO₂ 101 | | Reaktor 10 |

104

113

107

114

Methanol-
Wasser-
Gemisch 108

112

110

111

I1, I2, ...

Fig. 2

Fig. 3

H2O

700

E1(=)

105

60

52

62    61

51

50

$CO_2 + H_2$

$\downarrow$AS

Reaktion  106

107

Methanol-
Wasser-
Gemisch    108

Fig. 4

61

63

65

64

Fig. 5

61

E1(=)

10

CO$_2$

63

65

64

Fig. 6

60

**Europäisches Patentamt**
**European Patent Office**
**Office européen des brevets**

## EUROPÄISCHER RECHERCHENBERICHT

Nummer der Anmeldung

EP 11 15 5310

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 26 39 187 A1 (BRAUS KARL DR) 23. März 1978 (1978-03-23) * Seite 4, Zeile 1 - Seite 5, Absatz 1 * * Seite 6, Zeile 14 - Zeile 16; Ansprüche; Abbildungen * ----- | 1,2,5-17 | INV. B01J19/00 C01B3/04 C01B3/38 C07C29/152 C07C31/04 C10G2/00 C10G3/00 |
| X | WO 2007/002371 A2 (PRICE LESLIE DEAN [US]; GALLOWAY TERRY R [US]; PERK H F WILLIAM [US]) 4. Januar 2007 (2007-01-04) * Zusammenfassung * * Absatz [0023] - Absatz [0024] * * Absatz [0033] - Absatz [0037] * * Absatz [0040] - Absatz [0042] * * Absatz [0045]; Ansprüche; Abbildungen * ----- | 1-17 | |
| X | US 2003/065042 A1 (SHAW JOHN M [CA]) 3. April 2003 (2003-04-03) * Zusammenfassung * * Absatz [0006] * * Absatz [0008] - Absatz [0009] * * Absatz [0072] - Absatz [0080]; Ansprüche; Abbildungen 1, 2 * ----- | 1-17 | |
| X | US 4 776 171 A (PERRY JR JOHN H [US] ET AL) 11. Oktober 1988 (1988-10-11) * Zusammenfassung * * Spalte 4, Zeile 48 - Spalte 7, Zeile 28 * * Spalte 9, Zeile 30 - Zeile 44; Ansprüche; Abbildungen 1, 2 * ----- | 1-17 | RECHERCHIERTE SACHGEBIETE (IPC) B01J C01B C07C C10G |
| X | US 2008/058434 A1 (TONKOVICH ANNA LEE Y [US] ET AL) 6. März 2008 (2008-03-06) * Zusammenfassung * * Absatz [0002] - Absatz [0004] * * Absatz [0042] - Absatz [0049] * * Absatz [0080] - Absatz [0083]; Ansprüche; Abbildungen * ----- -/-- | 1-9, 11-14 | |

Der vorliegende Recherchenbericht wurde für alle Patentansprüche erstellt

| Recherchenort | Abschlußdatum der Recherche | Prüfer |
|---|---|---|
| München | 22. Februar 2012 | Nazario, Luis |

EPO FORM 1503 03.82 (P04C03)

Europäisches
Patentamt

European
Patent Office

Office européen
des brevets

## EUROPÄISCHER RECHERCHENBERICHT

**Nummer der Anmeldung**

EP 11 15 5310

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | JP 7 253025 A (YAMAGUCHI TAKASHI) 3. Oktober 1995 (1995-10-03) * Zusammenfassung; Abbildung * ----- | 15-17 | |
| A | BAYRAKTAR ET AL: "An experimental study on the performance parameters of an experimental CI engine fueled with diesel-methanol-dodecanol blends", FUEL, IPC SCIENCE AND TECHNOLOGY PRESS, GUILDFORD, GB, Bd. 87, Nr. 2, 15. November 2007 (2007-11-15), Seiten 158-164, XP022346675, ISSN: 0016-2361, DOI: 10.1016/J.FUEL.2007.04.021 * Zusammenfassung * * Seite 158, rechte Spalte, letzter Absatz - Seite 159, rechte Spalte, Absatz 1 * ----- | 1-17 | |
| A | EP 0 539 244 A1 (MITSUBISHI HEAVY IND LTD [JP]) 28. April 1993 (1993-04-28) * Zusammenfassung * * Spalte 2, Zeile 11 - Spalte 3, Zeile 27; Ansprüche; Abbildungen * ----- | 1-17 | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| München | 22. Februar 2012 | Nazario, Luis |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

........................................................

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 11 15 5310

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

22-02-2012

| Im Recherchenbericht angeführtes Patentdokument | Datum der Veröffentlichung | Mitglied(er) der Patentfamilie | Datum der Veröffentlichung |
|---|---|---|---|
| DE 2639187 A1 | 23-03-1978 | DE 2639187 A1<br>JP 53029437 A | 23-03-1978<br>18-03-1978 |
| WO 2007002371 A2 | 04-01-2007 | EP 1913683 A2<br>KR 20080059142 A<br>WO 2007002371 A2 | 23-04-2008<br>26-06-2008<br>04-01-2007 |
| US 2003065042 A1 | 03-04-2003 | AU 2002328724 A1<br>CA 2357527 A1<br>US 2003065042 A1<br>US 2009012332 A1<br>US 2010240778 A1 | 14-04-2003<br>01-04-2003<br>03-04-2003<br>08-01-2009<br>23-09-2010 |
| US 4776171 A | 11-10-1988 | CA 1334979 C<br>US 4776171 A | 28-03-1995<br>11-10-1988 |
| US 2008058434 A1 | 06-03-2008 | AU 2007293066 A1<br>CA 2662290 A1<br>CN 101528337 A<br>EP 2073917 A2<br>JP 2010502432 A<br>US 2008058434 A1<br>WO 2008030467 A2 | 13-03-2008<br>13-03-2008<br>09-09-2009<br>01-07-2009<br>28-01-2010<br>06-03-2008<br>13-03-2008 |
| JP 7253025 A | 03-10-1995 | JP 2627396 B2<br>JP 7253025 A | 02-07-1997<br>03-10-1995 |
| EP 0539244 A1 | 28-04-1993 | AU 656614 B2<br>AU 2213192 A<br>CA 2077880 A1<br>EP 0539244 A1<br>JP 5065237 A | 09-02-1995<br>11-03-1993<br>11-03-1993<br>28-04-1993<br>19-03-1993 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2010064948 W
- EP 11152947 A
- EP 0790226 B1
- WO 2010037441 A1
- EP 4483919 A2
- WO 2010069622 A1